# EUROPEAN PATENT SPECIFICATION

(11) **EP 4 599 874 B1**
(45) Date of publication and mention of the grant of the patent: **28.10.2026**
(21) Application number: 25185526.8
(22) Date of filing: 03.11.2022
(51) Int. Cl.: A61M 16/00

(54) **GAS DELIVERY SYSTEM**
GASABGABESYSTEM
SYSTÈME DE DISTRIBUTION DE GAZ

(30) Priority: 10.11.2021 EP 21207640
(43) Date of publication of application: 13.08.2025
(62) Document number(s) of the earlier application(s) in accordance with Art. 76 EPC: 22813944.0 / 4 429 742
(73) Proprietor: Philip Morris Products S.A., 2000 Neuchâtel (CH)
(72) Inventor: CHUA, Chin Suan, 159471 Redhill (SG); KHALID BIN ABDUL HALID, Danial, 159471 Redhill (SG); KRISHNAN, Subash, 159471 Redhill (SG); LAM, Sharon Ai Er, 159471 Redhill (SG); LEE, Tom, 159471 Redhill (SG); TAN, Wei Teck, 159471 Redhill (SG); TUNG, Ching Keong, 159471 Redhill (SG); XIA, Wenhao, 159471 Redhill (SG)
(74) Representative: Reddie & Grose LLP

(56) References cited:
- WO-A1-2021/195449
- US-A1- 2021 322 707
- US-B1- 6 474 334

## Description

The present disclosure relates to a gas delivery system, and to a method (not claimed) of using a gas delivery system.

Gas delivery systems are used to deliver gases or aerosols to subjects during pre-clinical *in vivo* testing. There are a limited number of commercially available gas delivery systems that are suitable for such testing. However, these commercially available systems are typically not efficient for tests with a large number of subjects and typically do not allow the system operator to choose between intra-tracheal and nasal delivery of gas to subjects.

WO 2021/195449 A1 discloses a ventilation apparatus comprising modular components to provide time controlled adaptive ventilation to one or more individuals simultaneously using one gas source and multiple valves proximal to the patient such as to provide individualized ventilation. The modular components can be easily manufactured and assembled in flexible configurations in any number of ways.

The present invention aims to provide an improved gas delivery system. In particular, the present invention aims to solve one or more of the problems set out above.

Thus, there is provided a gas delivery system for delivering gas to at least one subject. The system may comprise an upstream portion. The upstream portion may comprise a gas inlet. The system may comprise a downstream portion. The downstream portion may comprise a gas outlet. The system may comprise an intermediate portion. The intermediate portion may comprise a first gas delivery outlet. The upstream portion may be upstream of the intermediate portion. The intermediate portion may be upstream of the downstream portion. A gas flow path may be formed from the gas inlet of the upstream portion to the gas outlet of the downstream portion. A first gas delivery flow path may be formed from the gas inlet of the upstream portion to the first gas delivery outlet of the intermediate portion.

According to a first aspect of the disclosure and according to the invention, there is provided a gas delivery system for delivering gas to at least one subject. The system comprises an upstream portion comprising a gas inlet. The system comprises a downstream portion comprising a gas outlet. The system comprises an intermediate portion comprising a first gas delivery outlet. The upstream portion is upstream of the intermediate portion, and the intermediate portion is upstream of the downstream portion. A gas flow path is formed from the gas inlet of the upstream portion to the gas outlet of the downstream portion. A first gas delivery flow path is formed from the gas inlet of the upstream portion to the first gas delivery outlet of the intermediate portion. The system comprises an exhaust unit for providing the gas flow from the gas inlet to the gas outlet, the exhaust unit being configured to provide suction downstream of the gas outlet.

The gas delivery system may be a modular gas delivery system. The upstream portion may comprise or be an upstream module. The downstream portion may comprise or be a downstream module. The intermediate portion may comprise or be an intermediate module assembly. The intermediate module assembly may comprise a first intermediate module. The first intermediate module may comprise the first gas delivery outlet. The intermediate module assembly may be engageable with, and disengageable from, the upstream module. The intermediate module assembly may be engageable with, and disengageable from, the downstream module. The system, for example the upstream module, the downstream module and the intermediate module assembly, may be configured to be assembled into a first system configuration in which the upstream module is upstream of, and engaged with, the intermediate module assembly, and the intermediate module assembly is upstream of, and engaged with, the downstream module. In the first system configuration, the gas flow path may be formed from the gas inlet of the upstream module to the gas outlet of the downstream module. In the first system configuration, the first gas delivery flow path may be formed from the gas inlet of the upstream module to the first gas delivery outlet of the first intermediate module.

Thus, according to a second aspect of the disclosure, there is provided a modular gas delivery system for delivering gas to at least one subject. The system comprises an upstream module comprising a gas inlet, a downstream module comprising a gas outlet, and an intermediate module assembly comprising a first intermediate module, the first intermediate module comprising a first gas delivery outlet. The intermediate module assembly is engageable with, and disengageable from, the upstream module, and engageable with, and disengageable from, the downstream module. The upstream module, the downstream module and the intermediate module assembly are configured to be assembled into a first system configuration. In the first system configuration, the upstream module is upstream of, and engaged with, the intermediate module assembly, the intermediate module assembly is upstream of, and engaged with, the downstream module, a gas flow path is formed from the gas inlet of the upstream module to the gas outlet of the downstream module, and a first gas delivery flow path is formed from the gas inlet of the upstream module to the first gas delivery outlet of the first intermediate module.

Advantageously, the gas delivery system is modular. This may allow the system to take up less space when being stored or transported. In addition, if a module of the system is damaged, this may allow one to replace only the damaged module rather than the whole system.

The system may be for delivering gas to at least one subject, for example a plurality of subjects. The or each subject may be a non-human animal such as a mouse. Thus, the system may be for delivering gas to at least one non-human animal, for example a plurality of non-human animals. The system may be for delivering gas to a plurality of subjects, for example a plurality of non-human animals, simultaneously. As used herein, the term "subject" may be used to refer to a non-human animal.

Advantageously, the system may be for delivering gas to a plurality of subjects simultaneously. This may make the system more efficient for tests in which gas must be delivered to a plurality of subjects.

The first system configuration may be an operational configuration. That is, when the system is in the first system configuration, the system may be useable to deliver gas to at least one subject. In use, gas may be conveyed from the gas inlet, along the first gas delivery flow path, through the first gas delivery outlet, and then to the subject. Such gas may be referred to as delivery gas. Simultaneously, gas may be conveyed from the gas inlet, along the gas flow path, and through the gas outlet of the downstream module. Such gas may be referred to as exhaust gas.

In the first system configuration, the gas flow path may extend through at least a portion of the intermediate module assembly. In the first system configuration, the gas flow path may extend through at least a portion of the first intermediate module.

In the first system configuration, the upstream module, the first intermediate module, and the downstream module may be aligned, for example in a longitudinal direction. In the first system configuration, the upstream module, the intermediate module assembly, and the downstream module may be aligned, for example in a longitudinal direction. Advantageously, aligning various modules of the system may allow those modules to be supported on the same surface. In addition, aligning various modules of the system may allow the system to take up less space in use than if the modules were not aligned.

The intermediate module assembly may comprise a second intermediate module. The system may be useable with or without the second intermediate module. Advantageously, the provision of a second intermediate module may allow the system to accommodate more subjects at once, and therefore be more efficient for delivering gas to a large number of subjects.

The second intermediate module may be engageable with, and disengageable from, the first intermediate module. The first intermediate module may be engageable with, and disengageable from, the upstream module. The second intermediate module may be engageable with, and disengageable from, the upstream module. The second intermediate module may be engageable with, and disengageable from, the downstream module. Advantageously, this may allow the second intermediate module to be used in place of the first intermediate module in the first system configuration, for example should the first intermediate module be damaged.

The second intermediate module may comprise a second gas delivery outlet. Advantageously, where both the first intermediate module and the second intermediate module are used simultaneously, this may allow gas to be delivered simultaneously to a first subject via the first gas delivery outlet and to a second subject via the second gas delivery outlet.

The second intermediate module may be structurally identical to the first intermediate module. The second intermediate module may be structurally identical to the first intermediate module such that the first intermediate module and the second intermediate module may be used interchangeably in the system. Advantageously, this may allow the second intermediate module to be used in place of the first intermediate module in the first system configuration, for example should the first intermediate module be damaged.

The system may be configured to be assembled into a second system configuration. The second system configuration may be an operational configuration. That is, when the system is in the second system configuration, the system may be useable to deliver gas to at least one subject.

In the second system configuration, the upstream module may be upstream of the intermediate module assembly. In the second system configuration, the upstream module may be engaged with the intermediate module assembly. In the second system configuration, the upstream module may be upstream of one or both of the first intermediate module and the second intermediate module. In the second system configuration, the intermediate module assembly may be upstream of the downstream module. In the second system configuration, the intermediate module assembly may be engaged with the downstream module. In the second system configuration, one or both of the first intermediate module and the second intermediate module may be upstream of the downstream module.

In the second system configuration, a gas flow path may be formed from the gas inlet of the upstream module to the gas outlet of the downstream module.

In the second system configuration, a first gas delivery flow path may be formed from the gas inlet of the upstream module to the first gas delivery outlet of the first intermediate module.

In the second system configuration, a second gas delivery flow path may be formed from the gas inlet of the upstream module to the second gas delivery outlet of the second intermediate module.

In the second system configuration, the first intermediate module and the second intermediate module may be arranged in series. In the second system configuration, all of the intermediate modules of the intermediate module assembly may be arranged in series. Such configurations may be referred to as series system configurations.

In the second system configuration, the upstream module may be upstream of, and engaged with, the first intermediate module.

In the second system configuration, the first intermediate module may be upstream of the second intermediate module. In the second system configuration, the first intermediate module may be engaged with the second intermediate module. In the second system configuration, the first intermediate module may be coupled to the second intermediate module via one or more additional intermediate modules of the intermediate module assembly. The one or more additional intermediate modules may be located downstream of the first intermediate module. The one or more additional intermediate modules may be located upstream of the second intermediate module.

Advantageously, the number of intermediate modules being used in the second system configuration may be varied to suit the number of subjects being used in a given test.

In the second system configuration, the second intermediate module may be upstream of the downstream module. In the second system configuration, the second intermediate module may be engaged with the downstream module.

In the second system configuration, the gas flow path may extend through at least a portion of the intermediate module assembly. In the second system configuration, the gas flow path may extend through at least a portion of one or both of the first intermediate module and the second intermediate module. In the second system configuration, the gas flow path may extend through at least a portion of the first intermediate module and then, further downstream, through at least a portion of the second intermediate module.

In the second system configuration, the second gas delivery flow path may extend through at least a portion of the intermediate module assembly. In the second system configuration, the second gas delivery flow path may extend through at least a portion of the first intermediate module.

In the second system configuration, the upstream module and the intermediate module assembly may be aligned, for example in a longitudinal direction. In the second system configuration, the upstream module and the first intermediate module may be aligned, for example in a longitudinal direction. In the second system configuration, the first intermediate module and the second intermediate module may be aligned, for example in a longitudinal direction. In the second system configuration, the second intermediate module and the downstream module may be aligned, for example in a longitudinal direction. In the second system configuration, the intermediate module assembly and the downstream module may be aligned, for example in a longitudinal direction.

Advantageously, aligning the intermediate modules of the intermediate module assembly in a longitudinal direction may line up the gas delivery outlets of the intermediate modules in a row. This may make coupling subjects to the gas delivery outlets quicker and easier.

In the second system configuration, the first gas delivery outlet may be aligned with the second gas delivery outlet, for example in a longitudinal direction. Advantageously, this may make coupling subjects to the gas delivery outlets quicker and easier.

In the second system configuration, the first gas delivery outlet may be spaced from the second gas delivery outlet by at least 100, 150 or 200 millimetres, for example in a longitudinal direction. Advantageously, a greater spacing between gas delivery outlets may allow larger subjects to be coupled to the system. Alternatively, or in addition, a greater spacing between gas delivery outlets may allow easier handling of the subjects, for example during coupling to, and decoupling from, the system.

In the second system configuration, the first gas delivery outlet may be spaced from the second gas delivery outlet by less than 1,000, 750, 500, or 300 millimetres, for example in a longitudinal direction. Advantageously, a smaller spacing between gas delivery outlets may allow more subjects to be coupled to the system in a given space. In addition, it may be advantageous to minimise the lengths of the intermediate modules (and thus use a smaller spacing between gas delivery outlets of an intermediate module) because, where the system is used to deliver an aerosol, the concentration of the aerosol in the system may be less spatially homogeneous across a longer path length, especially if the aerosol flow rate or pressure in the system is low.

In the second system configuration, the first intermediate module and the second intermediate module may be arranged in parallel. Such a configuration may be referred to as a parallel system configuration.

In the second system configuration, the upstream module may be engaged with multiple intermediate modules, for example each intermediate module, of the intermediate module assembly. In the second system configuration, the upstream module may be engaged with one or both of the first intermediate module and the second intermediate module.

In the second system configuration, the first intermediate module may be neither upstream nor downstream of the second intermediate module. In the second system configuration, each intermediate module may be neither upstream nor downstream of every other intermediate module.

In the second system configuration, multiple intermediate modules, for example each intermediate module, of the intermediate module assembly may be engaged with the downstream module. In the second system configuration, one or both of the first intermediate module and the second intermediate module may be engaged with the downstream module.

In the second system configuration, multiple gas flow paths may be formed from the gas inlet of the upstream module, through at least a portion of the intermediate module assembly, and optionally to the gas outlet of the downstream module. In the second system configuration, a first gas flow path may be formed from the gas inlet of the upstream module, through first intermediate module, and optionally to the gas outlet of the downstream module. In the second system configuration, a second gas flow path may be formed from the gas inlet of the upstream module, through second intermediate module, and optionally to the gas outlet of the downstream module.

In the second system configuration, the system may define one or more gas flow paths arranged in parallel, these gas flow paths extending from the gas inlet of the upstream module, through multiple intermediate modules of the intermediate module assembly, and optionally to the gas outlet of the downstream module. Advantageously, gas flow paths arranged in parallel may extend through multiple intermediate modules. This may allow the system to be coupled to more subjects simultaneously.

In the second system configuration, a first gas delivery flow path may be formed from the gas inlet of the upstream module to the first gas delivery outlet of the first intermediate module. In the second system configuration, a second gas delivery flow path may be formed from the gas inlet of the upstream module to the second gas delivery outlet of the second intermediate module. In the second system configuration, the second gas delivery flow path may not extend through the first intermediate module.

In the second system configuration, the upstream module and the intermediate module assembly may be aligned, for example in a longitudinal direction. In the second system configuration, multiple intermediate modules, for example each intermediate module, of the intermediate module assembly may be aligned, for example in a transverse direction. The transverse direction may be substantially perpendicular to the longitudinal direction. In the second system configuration, the first intermediate module and the second intermediate module may be aligned, for example in a transverse direction. In the second system configuration, the intermediate module assembly and the downstream module may be aligned, for example in a longitudinal direction.

Advantageously, aligning the intermediate modules of the intermediate module assembly in a transverse direction may allow the provision of substantially opposing gas delivery outlets on either side of the system. This may allow subjects to be coupled to the system on either side of the system, thus allowing more subjects to be coupled to the system than would otherwise be possible.

In the second system configuration, the first gas delivery outlet may be aligned with the second gas delivery outlet, for example in a transverse direction.

The system may be configured to be assembled into a series system configuration, for example in which the first intermediate module and the second intermediate module are arranged in series, and also configured to be assembled into a parallel system configuration, for example in which the first intermediate module and the second intermediate module are arranged in parallel.

Advantageously, a system which is configurable into either a series system configuration or a parallel system configuration may provide the system operator with more flexibility to accommodate more subjects.

The series system configuration may be the second system configuration described above. The parallel system configuration may be the second system configuration described above. Thus, features described above in relation to the second system configuration may be applicable to one or both of the series system configuration and the parallel system configuration.

The system may comprise one or more adaptors, for example one or both of an upstream adaptor and a downstream adaptor. The adaptor or adaptors may allow the system to be assembled into the series system configuration or the parallel system configuration.

The upstream adaptor may be engageable with, and disengageable from, the upstream module. The upstream adaptor may be engageable with, and disengageable from, the intermediate module assembly. In any operational system configuration, the upstream adaptor may be downstream of the upstream module and upstream of the intermediate module assembly. In the parallel system configuration, the upstream adaptor may divide a gas flow path from the upstream module into two or more gas flow paths. Advantageously, such a parallel system configuration may allow more subjects to be coupled to the system in a given space.

The downstream adaptor may be engageable with, and disengageable from, the downstream module. The downstream adaptor may be engageable with, and disengageable from, the intermediate module assembly. In any operational system configuration, the downstream adaptor may be downstream of the intermediate module assembly and upstream of the downstream module. In the parallel system configuration, the downstream adaptor may merge two or more gas flow path from the intermediate module assembly into fewer gas flow paths, for example a single gas flow path. Advantageously, this may mean that only a single downstream module is required. This may reduce a cost of the system, particularly where the downstream module comprises or is engaged with an exhaust unit for providing or allowing gas flow through the system, or a pressure adjustment mechanism for adjusting a pressure in the system.

The system may be configured to deliver gas to a first subject or a first plurality of subjects by intra-tracheal delivery. The system may be configured to deliver gas to a second subject or a second plurality of subjects by nasal delivery. The system may be configured to simultaneously deliver gas to at least one subject by intra-tracheal delivery and at least one subject by nasal delivery.

Advantageously, the system may allow gas to be delivered by intra-tracheal delivery, by nasal delivery, or by both intra-tracheal delivery and nasal delivery simultaneously. This provides flexibility to the system operator when deciding how to deliver gas to subjects.

The system may comprise an intra-tracheal delivery component. The system may comprise a nasal delivery component. The intra-tracheal delivery component may be engageable with, and disengageable from, one or both of the first gas delivery outlet and the second gas delivery outlet so as to allow delivery of gas to the first subject by intra-tracheal delivery. The intra-tracheal delivery component may be for intubating a subject. The nasal delivery component may be engageable with, and disengageable from, the other or both of the first gas delivery outlet and the second gas delivery outlet so as to allow delivery of gas to the second subject by nasal delivery. The nasal delivery component may be for accommodating, or containing, a subject.

Advantageously, each gas delivery outlet may be configured to be engageable with, and disengageable from, the intra-tracheal delivery component and the nasal delivery component. This may allow the system operator to decide which delivery component to engage with each gas delivery outlet.

The first intermediate module comprises a first additional gas delivery outlet. In one or both of the first system configuration and the second system configuration, or indeed in any operational system configuration, an additional gas delivery flow path may be defined from the gas inlet of the upstream module to the first additional gas delivery outlet of the first intermediate module. Advantageously, this may allow gas to be delivered to two subjects via the first intermediate module.

The first additional gas delivery outlet may be aligned with the first gas delivery outlet, for example in a longitudinal direction. Advantageously, the first additional gas delivery outlet being aligned with the first gas delivery outlet may result in these outlets being lined up in a row so as to make coupling subjects to the outlets quicker and easier.

The first additional gas delivery outlet may be spaced from the first gas delivery outlet by at least 100, 150, or 200 millimetres, for example in a longitudinal direction. Advantageously, a greater spacing between gas delivery outlets may allow larger subjects to be coupled to the system. Alternatively, or in addition, a greater spacing between gas delivery outlets may allow easier handling of the subjects, for example during coupling to, and decoupling from, the system.

The first additional gas delivery outlet may be spaced from the first gas delivery outlet by less than 1,000, 750, 500 or 300 millimetres, for example in a longitudinal direction. Advantageously, a smaller spacing between gas delivery outlets may allow more subjects to be coupled to the system in a given space.

The first intermediate module may comprise a body. The body may be defined at least in part by an outer wall. The body may be substantially tubular. The first intermediate module may define an internal lumen. The internal lumen may be defined, at least in part, by the body of the first intermediate module. The internal lumen may extend in a longitudinal direction. The gas flow path may extend through the internal lumen. The second gas delivery flow path may extend through the internal lumen. The second gas delivery flow path may extend partly through the internal lumen.

The first intermediate module may comprise a first gas delivery outlet structure. At least a portion of the first gas delivery outlet structure may define the first gas delivery outlet. At least a portion of the first gas delivery outlet structure may be upstream of the first gas delivery outlet. The first gas delivery outlet structure may protrude outwardly from the outer wall of the first intermediate module. Advantageously, the first gas delivery outlet structure may make it easier to couple a subject to the first intermediate module.

The first gas delivery outlet structure may be engageable with an intra-tracheal delivery component. The first gas delivery outlet structure may be engageable with a nasal delivery component. The first gas delivery outlet structure may be configured such that it is engageable with either an intra-tracheal delivery component or a nasal delivery component.

The first gas delivery outlet structure may protrude from the outer wall in a first gas delivery outlet structure direction. The longitudinal direction and the first gas delivery outlet structure direction may be non-parallel. An angle between the longitudinal direction and the first gas delivery outlet structure direction may be at least 15 or 30 degrees. An angle between the longitudinal direction and the first gas delivery outlet structure direction may be no more than 90 or 75 degrees. This angle may be measured between the first gas delivery outlet and a point in the internal lumen located in the gas flow path and downstream of an entrance region of the first gas delivery outlet structure.

Advantageously, the angle being at least 15 or 30 degrees may mean that there is more space for a subject coupled to the first gas delivery outlet. This is because the subject will be directed away from the module, rather than parallel with the module.

At least a portion of the first intermediate module, for example least a portion of the body or the outer wall, may be transparent or translucent. Advantageously, this may allow a system operator to see within the intermediate module. In the case of a visible gas flow, this may allow a system operator to see the gas flow within the first intermediate module.

At least a portion of the first intermediate module, for example least a portion of the body or the outer wall, may be formed from glass, for example transparent or translucent glass. Advantageously, glass is relatively rigid, inert, and may be transparent or translucent.

A first portion, for example a first end, of the first intermediate module may comprise a first engagement means. A second portion, for example a second end, of the first intermediate module may comprise a second engagement means. The first end and the second end may be opposite ends of the first intermediate module. The internal lumen may be defined from the first end to the second end. Each intermediate module may comprise first and second engagement means, similar to the first intermediate module. The first engagement means of any particular intermediate module may be configured to engage with the second engagement means of any other intermediate module. Each first engagement means may be one of a male engagement means and a female engagement means, and each second engagement means may be the other of the male engagement means and female engagement means. The male and female engagement means may be configured to engage with each other, for example so as to engage the modules with each other.

As an example, the first engagement means may comprise or be an internal screw thread, and the second engagement means may comprise or be an external screw thread. In use, two intermediate modules may be engaged by engaging the internal screw thread of one intermediate module with the external screw thread of the other intermediate module. However, other engagement means are possible. For example, a snap-fit connection, or interference fit, between modules could be used.

Advantageously, this may allow each intermediate module to be engaged with any other intermediate module.

The upstream module may comprise an upstream module engagement means for engaging one or either of the first and second engagement means of an intermediate module. The downstream module may comprise a downstream module engagement means for engaging the other or either of the first and second engagement means of an intermediate module. Features described in relation to the engagement means of the intermediate modules may be applicable to one or both of the upstream module engagement means and the downstream module engagement means.

The upstream module engagement means may be one of a male or female engagement means, and the downstream module engagement means may be the other of a male and female engagement means. As an example, the upstream module engagement means may comprise or be an internal screw thread, and the downstream module engagement means may comprise or be an external screw thread.

Advantageously, this may allow any intermediate module to engage with the upstream module or the downstream module.

One or both of the upstream module engagement means and the downstream module engagement means may comprise a clamping surface. One or both of the first engagement means and the second engagement means of one or more intermediate modules, for example the first intermediate module, the second intermediate module or each intermediate module, may comprise a clamping surface. The gas delivery system, for example any engagement means of the gas delivery system, may comprise at least one clamp. The gas delivery system may comprise at least one clamp for each pair of clamping surfaces of the system.

In use, the clamp or clamps may clamp adjacent clamping surfaces of the modules of the system. For example, in a configuration such as the second system configuration, a first clamp may clamp the clamping surface of the upstream module engagement means to the clamping surface of the first engagement means of the first module, a second clamp may clamp the clamping surface of the second engagement means of the first module to the clamping surface of the first engagement means of the second intermediate module, and a third clamp may clamp the clamping surface of the second engagement means of the second intermediate module to the clamping surface of the downstream module engagement means. Thus, the clamp or clamps may engage various modules with one another.

Advantageously, clamps may provide a secure and reliable way to engage modules with one another.

The upstream module, or a component engageable with the upstream module, may be configured to provide gas flow through the gas inlet at a predetermined pressure. The upstream module, or a component engageable with the upstream module, may be configured to provide gas flow through the gas inlet at a pressure above atmospheric pressure. This pressure may be at least 10 or 30 Pascals above atmospheric pressure. This pressure may be no more than 50 or 30 Pascals above atmospheric pressure. This pressure may be between 0 and 50, for example between 10 and 30, Pascals above atmospheric pressure. This pressure may drive gas flow through the system. That is, this pressure may drive one or more of the gas flow along the gas flow path, the first delivery gas flow along the first delivery gas flow path, and the second delivery gas flow along the second delivery gas flow path. Advantageously, where the gas delivery system is used to deliver an aerosol, a pressure greater than atmospheric pressure may allow the system to provide a more spatially homogenous aerosol concentration through the system.

The upstream module may comprise a first inlet port for introducing a first substance into a gas flow from the gas inlet. The first substance may be an aerosol, or may form an aerosol once entrained in the gas flow from the gas inlet. Advantageously, the first inlet port may allow a system operator to introduce a first substance, such as an aerosol or gaseous drug, into the gas flow, and thereby deliver the first substance to one or more subjects coupled to the system.

The upstream module may comprise a second inlet port for introducing a second substance into the gas flow from the gas inlet. Advantageously, the second inlet port may allow a system operator to introduce a second substance, such as a gaseous anaesthetic, into the gas flow, and thereby deliver the second substance to one or more subjects coupled to the system. It may be particularly advantageous for the upstream module to comprise both the first inlet port and the second inlet port as this may allow a system operator to deliver two substances, for example a gaseous drug and a gaseous anaesthetic, to each subject simultaneously.

The system may comprise a pressure sensor for determining a pressure in the system, such as a manometer. The downstream module may comprise the pressure sensor. The pressure sensor may determine a pressure in the downstream module. The pressure sensor may comprise or be a pressure gauge, such as a manometer, for determining and displaying the pressure in the system. Advantageously, a pressure sensor may allow a system operator to ensure that the system is operating at an optimal pressure.

The system may comprise a pressure adjustment mechanism for adjusting a pressure in the system, for example in the intermediate module assembly or in the first or second intermediate modules. Advantageously, this may allow a system operator to run tests at different pressures in the system. As described in more detail in relation to the fourth aspect of this disclosure, the system may comprise the pressure adjustment mechanism even if the system is not a module system.

The system may comprise a pressure adjustment mechanism for adjusting a pressure in the system during use of the system to deliver gas to one or more subjects. It may be particularly advantageous to be able to adjust the pressure in the system during use of the system to deliver gas to one or more subjects. This may allow a system operator to run tests in which the pressure is varied during the test.

The downstream module may comprise the pressure adjustment mechanism.

The pressure adjustment mechanism may be a manual pressure adjustment mechanism configured to allow a user to manually set a pressure for the system, for example prior to use of the system to deliver gas to one or more subjects. The pressure adjustment mechanism may be a manual pressure adjustment mechanism configured to allow a user to manually adjust a pressure in the system, for example during use of the system to deliver gas to one or more subjects. Advantageously, a manual pressure adjustment mechanism may be reliable and straightforward to operate.

The pressure adjustment mechanism may be an automatic pressure adjustment mechanism configured to automatically adjust a pressure in the system, for example during use of the system to deliver gas to one or more subjects. Advantageously, an automatic pressure adjustment mechanism may allow the pressure to be adjusted more precisely than a manual pressure adjustment mechanism.

The pressure adjustment mechanism may be a hybrid pressure adjustment mechanism. The hybrid pressure adjustment mechanism may be configurable between a manual state and an automatic state. In the manual state, the hybrid pressure adjustment mechanism may allow a user to manually set a pressure for the system, for example prior to use of the system to deliver gas to one or more subjects. In the automatic state, the hybrid pressure adjustment mechanism may automatically adjust a pressure in the system, for example during use of the system to deliver gas to one or more subjects.

During use of the system to deliver gas to one or more subjects, the pressure adjustment mechanism may be configured maintain a pressure in the system, for example in the intermediate module assembly, or in one or both of the first intermediate module and the second intermediate module, within a predetermined pressure range. Advantageously, this may allow greater control over gas flow rates through the system, and thereby provide more consistent gas delivery to subjects.

The pressure adjustment mechanism may be configured maintain the pressure within the predetermined pressure range based on a pressure sensed by the pressure sensor.

The predetermined pressure range may be greater than atmospheric pressure. The predetermined pressure range may have an upper limit no more than 50 or 30 Pascals above atmospheric pressure. The predetermined pressure range may have a lower limit of no less than 0 or 10 Pascals above atmospheric pressure. Thus, the predetermined pressure range may be between 0 and 50, or between 10 and 30, Pascals above atmospheric pressure. The pressures referred to in this paragraph refer to total pressure, rather than static pressure or dynamic pressure. Advantageously, where the system is used to deliver an aerosol to one or more subjects, maintaining the pressure in the system greater than atmospheric pressure may provide a more spatially homogenous aerosol concentration through the system. Further, the inventors have found that the specific pressure ranges noted above may provide spatially homogenous aerosol concentration through the system and allow optimal gas delivery flow rates through the gas delivery outlets of the intermediate modules.

The pressure adjustment mechanism may comprise a pressure adjustment component. The pressure adjustment component may define an opening. The pressure adjustment component may be moveable, for example during use of the system to deliver gas to one or more subjects, so as to vary a size of the opening. Advantageously, this may provide a straightforward and reliable method for adjusting the pressure in the system.

The pressure adjustment component may be moveable, for example during use of the system to deliver gas to one or more subjects, between a first position in which the opening has a first size, and a second position in which the opening has a second size, different to the first size.

The pressure adjustment component may comprise a plurality of blades. The plurality of blades may define the opening defined by the pressure adjustment component. At least one, for example each, of the plurality of blades may be moveable, for example during use of the system to deliver gas to one or more subjects, so as to vary a size of the opening. For example, movement of the blades may move the pressure adjustment component between the first position and the second position and vary the size of the opening between the first size and the second size. The gas flow path may extend through the opening. The gas outlet of the downstream module may comprise or may be the opening. Advantageously, the pressure adjustment component defining the opening, and the opening being the gas outlet of the downstream module, may provide a convenient method for adjusting pressure in the system.

The plurality of blades may be arranged in an iris arrangement. Each of the plurality of blades may be configured to move between a first blade position and a second blade position. When each of the plurality of blades is in the first blade position, the pressure adjustment component may be in the first position. When each of the plurality of blades is in the second blade position, the pressure adjustment component may be in the second position. Thus, movement of each of the blades from the first blade position to the second blade position may adjust the size of the opening from the first size to the second size.

In one or both of the first position and the second position of the pressure adjustment component, each blade of the plurality of blades may overlap at least one other blade of the plurality of blades. When each blade is in one or either of the first blade position and the second blade position, each blade of the plurality of blades may overlap at least one other blade of the plurality of blades.

The first size of the opening may be smaller than the second size of the opening. The opening may have a centre. Each of the plurality of blades may comprise a curved surface, for example a concave curved surface. The curved surfaces of the blades may together define the opening.

Each of the plurality of blades may be configured to move further from, for example in a radial direction directly away from, the centre of the opening to move the pressure adjustment component from the first position to the second position. Each of the plurality of blades may be configured to move further from, for example in a radial direction directly away from, the centre of the opening to move from the first blade position to the second blade position.

Each of the plurality of blades may be configured to move closer to, for example in a radial direction directly towards, the centre of the opening to move from the second blade position to the first blade position. Each of the plurality of blades may be configured to move closer to, for example in a radial direction directly towards, the centre of the opening to move from the pressure adjustment component from the second position to the first position.

Each of the plurality of blades may be configured to rotate to move the pressure adjustment component between the first position and the second position. Each of the plurality of blades may be configured to rotate to move between the first blade position and the second blade position.

The pressure adjustment component may be configured to move to a third position in which the opening has been closed. The plurality of blades may be configured to move to a third blade position to move the pressure adjustment component to the third position. Thus, the plurality of blades may be configured to move to the third blade position to close the opening. Advantageously, this may protect the system from ingress of contaminants or other debris, for example when the system is not in use.

The pressure adjustment component may be disassemblable and reassemblable. Advantageously, this may allow the pressure adjustment component to be disassembled and thoroughly cleaned after an experiment, then reassembled for the next experiment. Thoroughly cleaning the pressure adjustment component reduces the likelihood of the system being contaminated.

The pressure adjustment component may be engageable with, and disengageable from, the system, for example the downstream module. Advantageously, this may mean that, should the pressure adjustment component be damaged, it can be replaced. In addition, this may make disassembling and reassembling the pressure adjustment component easier as this can be done away from the rest of the system.

The pressure adjustment mechanism may comprise an iris diaphragm. An iris diaphragm is sometimes referred to as a camera aperture orifice. The skilled person would be aware of how an iris diaphragm operates. The pressure adjustment component may be or comprise the iris diaphragm. The iris diaphragm may define the opening. The iris diaphragm may comprise the plurality of blades. The blades of the iris diaphragm may be moveable as set out above. Advantageously, an iris diaphragm is easily disassemblable and reassemblable. This allows the iris diaphragm to be disassembled and thoroughly cleaned after an experiment, and then reassembled for the next experiment. Thoroughly cleaning the iris diaphragm may reduce the likelihood of the system being contaminated.

The pressure adjustment component may comprise a first plate. The first plate may comprise a first aperture. The pressure adjustment component may comprise a second plate. The second plate may comprise a second aperture. The first plate may be in contact with the second plate. In one or more positions of the first plate and the second plate, the first aperture and the second aperture may partially or completely align so as to define an opening extending through the first plate and the second plate.

The opening extending through the first plate and the second plate may be the opening defined by the pressure adjustment component described earlier. The gas flow path may extend through the opening. The gas outlet of the downstream module may comprise or may be the opening.

The first plate may be moveable, for example one or more of translatable and rotatable, relative to the second plate. The first plate may be moveable relative to the second plate during use of the system to deliver gas to one or more subjects. The first plate may be moveable relative to the second plate so as to vary a size of the opening. The first plate may be moveable relative to the second plate between a first position in which the opening has a first size, and a second position in which the opening has a second size, different to the first size.

Advantageously, such a pressure adjustment component may provide a straightforward and reliable method for adjusting the pressure in the system. Further, such a pressure adjustment component may be relatively simple to disassemble for cleaning and reassemble for re-use. Further still, such a pressure adjustment component may advantageously have relatively few moving parts and may therefore be less likely to malfunction than other, more complicated pressure adjustment components with more moving parts.

The first plate may comprise a first plurality of apertures. The first plurality of apertures may comprise the first aperture. The second plate may comprise a second plurality of apertures. The second plurality of apertures may comprise the second aperture. In one or more positions of the first plate and the second plate, multiple pairs of apertures may partially or completely align so as to define multiple openings extending through the first plate and the second plate, each pair of apertures comprising an aperture of the first plurality of apertures and an aperture of the second plurality of apertures. As would be understood by the skilled person after reading this disclosure, features described in relation to the opening of the pressure adjustment component may be applicable to each opening of the multiple openings described here.

The first plate may be moveable relative to the second plate so as to vary a size of each of the multiple openings. The first plate may be moveable relative to the second plate between a first position in which each of the multiple openings has a first size, and a second position in which each of the multiple openings has a second size, different to the first size.

During use, for example use of the system to deliver gas to one or more subjects, one of the first plate and the second plate may be held stationary. During use, only one of the first plate and the second plate may move relative to one or more other components of the system. For example, during use, only one of the first plate and the second plate may move relative to one or more of the upstream module, the intermediate module assembly and the downstream module. During use, one of the first plate and the second plate may be fixed relative to one or more other components of the system. For example, during use, one of the first plate and the second plate may be fixed relative to one or more of the upstream module, the intermediate module assembly and the downstream module. Advantageously, by providing relative movement between the first plate and the second plate with movement of only one of the first plate and the second plate, the number of moving parts of the pressure adjustment component may be minimised. This may mean that the pressure adjustment component is less likely to malfunction than other, more complicated pressure adjustment components with more moving parts.

The pressure adjustment mechanism may comprise a processor. In use, the processor may receive an input from the pressure sensor. In use, the processor may control movement of the pressure adjustment component, for example to adjust the size of the opening. Advantageously, the processor may be used to automate movement of the pressure adjustment component so as to adjust a pressure in the system, for example during use of the system to deliver gas to one or more subjects. In addition, the processor receiving an input from the pressure sensor may allow the processor to move the pressure adjustment component and receive real-time feedback on how the pressure in the system has consequently changed. This may allow more precise control of the pressure in the system.

In use, processor may use the input from the pressure sensor to maintain a pressure in the system, for example in the intermediate module assembly or in one or both of the first intermediate module and the second intermediate module, within a predetermined pressure range, for example one of the predetermined pressure ranges noted above.

In use, the processor may use the input from the pressure sensor to control movement of the pressure adjustment component to maintain a pressure in the system, for example in the intermediate module assembly or in one or both of the first intermediate module and the second intermediate module, within a predetermined pressure range, for example one of the predetermined pressure ranges noted above.

The system comprises an exhaust unit. The exhaust unit may be engageable with, and disengageable from, the system. The exhaust unit may be engageable with, and disengageable from, the downstream module. In one or both of the first system configuration and the second configuration, the exhaust unit may be located downstream of the downstream module. The exhaust unit is for providing the gas flow from the gas inlet to the gas outlet. In use, the exhaust unit provides suction downstream of the gas outlet. This may provide or allow the gas flow from the gas inlet to the gas outlet. The exhaust unit may ensure that gas from the gas inlet is ultimately vented in an appropriate location.

The system may comprise a spacer for spacing the exhaust unit from the downstream module, for example in a longitudinal direction. In one or both of the first system configuration and the second configuration, the spacer may be engaged with the downstream module and the exhaust unit may be engaged with the spacer. **In** one or both of the first system configuration and the second configuration, the spacer may be engaged with the downstream module and the exhaust unit may be engaged with the spacer, such that the spacer spaces the exhaust unit from the downstream module.

The spacer may be substantially tubular. The spacer may comprise a substantially tubular body. The spacer, or the tubular body of the spacer, may comprise a first open end and a second open end. The first open end may oppose the second open end. The spacer, or the tubular body of the spacer, may define an internal lumen, for example from the first open end to the second open end. The spacer, or the tubular body of the spacer, or the internal lumen of the spacer, may define a longitudinal flow path, for example from the first open end to the second open end. The longitudinal flow path may be located within the substantially tubular body. In one or both of the first system configuration and the second configuration, the spacer may be engaged with the downstream module and the exhaust unit may be engaged with the spacer, such that air flows from the intermediate module assembly through the internal lumen or longitudinal flow path.

The spacer may comprise one or more apertures. The one or more apertures may extend in a radial direction. The one or more apertures may extend radially through the substantially tubular body. The one or more apertures may extend from outside the spacer, or the substantially tubular body, to the internal lumen or longitudinal flow path. The one or more apertures may extend at least 2, 5, 10, 20, or 50 millimetres in a longitudinal direction. The one or more apertures may extend no more than 200, 150, 100, or 75 millimetres in a longitudinal direction.

In one or both of the first system configuration and the second system configuration, the spacer may be engaged with the downstream module and the exhaust unit may be engaged with the spacer, such that the exhaust unit is configured to draw air from the atmosphere through the one or more apertures, and simultaneously allow air from the gas inlet to flow through the system. The air drawn from the atmosphere may be different to the air flowing through the system, for example from the intermediate module assembly. That is, these may be two distinct flow paths. These flow paths may merge within the spacer, or substantially tubular body. Advantageously, the exhaust unit may allow the pressure within the system, for example within the intermediate module assembly, to remain stable even if the exhaust pressure were to fluctuate.

The spacer may be configured to space the exhaust unit from the downstream module by at least 2, 5, 10, 20, or 50 millimetres. For example, in an operational configuration, such as in one or both of the first system configuration and the second system configuration, the spacer may space the exhaust unit from the downstream module by at least 2, 5, 10, 20, or 50 millimetres.

The spacer may be configured to space the exhaust unit from the downstream module by no more than 200, 150, 100, 75, or 50 millimetres. For example, in an operational configuration, such as in one or both of the first system configuration and the second system configuration, the spacer may space the exhaust unit from the downstream module by no more than 200, 150, 100, 75, or 50 millimetres.

The inventors have found the above spacings and lengths of the at least one aperture to be particularly advantageous when used as part of the gas delivery system. This is because, if the spacing or length is too large, then gas from the gas delivery system, for example from the upstream module or intermediate module assembly of the system, may leak out of the at least one aperture during use. However, if the spacing is too small, then the exhaust pressure applied by the exhaust unit may influence the pressure within the gas delivery system, for example within the intermediate modules, more than is desirable. That is, if the spacing is too small, the pressure within the gas delivery system, for example within the intermediate modules may not remain stable if the exhaust pressure were to fluctuate.

Each intra-tracheal or nasal delivery component may comprise, or be engaged with, a sampler for sampling the gas or aerosol delivered through the intra-tracheal or nasal delivery component. Advantageously, this may give an accurate estimate of the doses of particular substances delivered to subjects through the delivery component.

One or more, for example each gas delivery outlet or gas delivery outlet structure may comprise, or be engaged with, a sampler for sampling the gas or aerosol delivered through the gas delivery outlet. Advantageously, this may give an accurate estimate of the doses of particular substances delivered to subjects through the gas delivery outlet.

The system, for example the upstream module, may comprise, or be engaged with, an upstream sampler for sampling the gas or aerosol flowing through or near the upstream module. The system, for example the downstream module, may comprise, or be engaged with, a downstream sampler for sampling the gas or aerosol flowing through or near the downstream module. It may be particularly advantageous for the system to comprise both the upstream sampler and the downstream sampler. This is because this may be used to determine whether the concentration of a particular substance, for example the aerosol concentration, is homogeneous across the length of the system.

The intermediate module assembly may comprise the first intermediate module and one or more further intermediate modules. The intermediate module assembly may comprise at least 1, 2, 3, 4, or 5 intermediate modules in addition to the first intermediate module. One of these additional intermediate modules may be the second intermediate module.

Features described in relation to the first intermediate module may be applicable to the second intermediate module, or to any other intermediate module, of the intermediate module assembly.

Features described in relation to the second intermediate module may be applicable to the first intermediate module, or to any other intermediate module, of the intermediate module assembly.

Each intermediate module of the intermediate module assembly may be engageable with, and disengageable from, every other intermediate module of the intermediate module assembly.

Each intermediate module of the intermediate module assembly may be engageable with, and disengageable from, the upstream module.

Each intermediate module of the intermediate module assembly may be engageable with, and disengageable from, the downstream module.

Each intermediate module of the intermediate module assembly may be structurally identical to every other intermediate module. Each intermediate module may be structurally identical to every other intermediate module such that the intermediate modules may be used interchangeably. Advantageously, this may allow any intermediate module to be used in place of another intermediate module, for example should any intermediate module be damaged.

At least one intermediate module of the intermediate module assembly may be for intra-tracheal delivery of gas to a first subject. That intermediate module may comprise, or be engageable or engaged with, an intra-tracheal delivery component for intra-tracheal delivery of gas to the first subject.

At least one intermediate module of the intermediate module assembly may be for nasal delivery of gas to a second subject. That intermediate module may comprise, or be engageable or engaged with, nasal delivery component for nasal delivery of gas to the second subject.

Advantageously, where the system comprises an intermediate module for intra-tracheal delivery and an intermediate module for nasal delivery, the system may be able to simultaneously deliver gas to a first subject by intra-tracheal delivery and deliver gas to a second subject by nasal delivery. Advantageously, this may allow a fairer test for comparing intra-tracheal delivery and nasal delivery since other variables, such as the concentration of any particular drug in the delivery gas flow, may be more likely to be constant in a single test allowing both types of delivery, than across two tests, each test allowing only one type of delivery.

There is also provided a method of using, or assembling, a system as described above, for example the system according to the second aspect. The method may comprise engaging the upstream module with the intermediate module assembly. The method may comprise engaging the downstream module with the intermediate module assembly. The method may comprise engaging the upstream module and the downstream module with the intermediate module assembly such that the upstream module is upstream of, and engaged with, the intermediate module assembly. The method may comprise engaging the upstream module and the downstream module with the intermediate module assembly such that the intermediate module assembly is upstream of, and engaged with, the downstream module. The method may comprise engaging the upstream module and the downstream module with the intermediate module assembly such that the gas flow path is formed from the gas inlet of the upstream module to the gas outlet of the downstream module. The method may comprise engaging the upstream module and the downstream module with the intermediate module assembly such that the first gas delivery flow path is formed from the gas inlet of the upstream module to the first gas delivery outlet of the first intermediate module.

Thus, according to a third aspect of the disclosure, there is provided a method of using, or assembling, a system as described above, for example the system according to the second aspect. The method comprises engaging the upstream module and the downstream module with the intermediate module assembly such that: the upstream module is upstream of, and engaged with, the intermediate module assembly; the intermediate module assembly is upstream of, and engaged with, the downstream module; the gas flow path is formed from the gas inlet of the upstream module to the gas outlet of the downstream module; and the first gas delivery flow path is formed from the gas inlet of the upstream module to the first gas delivery outlet of the first intermediate module.

The method may comprise or be a method of assembling the system into the first system configuration or the second system configuration.

Engaging the upstream module and the downstream module with the intermediate module assembly may comprise engaging an upstream end of the intermediate module assembly with the upstream module, for example a downstream end of the upstream module.

Engaging the upstream module and the downstream module with the intermediate module assembly may comprise engaging a downstream end of the intermediate module assembly with the downstream module, for example an upstream end of the downstream module.

Engaging the upstream module and the downstream module with the intermediate module assembly may comprise engaging the first intermediate module, for example an upstream end of the first intermediate module, with the upstream module, for example a downstream end of the upstream module.

Engaging the upstream module and the downstream module with the intermediate module assembly may comprise engaging the first intermediate module, for example a downstream end of the first intermediate module, with the downstream module, for example an upstream end of the downstream module.

The intermediate module assembly may comprise a second intermediate module, as described above. In this case, engaging the upstream module and the downstream module with the intermediate module assembly may comprise engaging the first intermediate module, for example an upstream end of the first intermediate module, with the upstream module, for example a downstream end of the upstream module. Also in this case, engaging the upstream module and the downstream module with the intermediate module assembly may comprise engaging the second intermediate module, for example an upstream end of the second intermediate module, with the first intermediate module, for example a downstream end of the first intermediate module. Also in this case, engaging the upstream module and the downstream module with the intermediate module assembly may comprise engaging the second intermediate module, for example a downstream end of the second intermediate module, with the downstream module, for example an upstream end of the downstream module.

The intermediate module assembly may comprise the first intermediate module, one or more additional intermediate modules, and a final intermediate module. Engaging the upstream module and the downstream module with the intermediate module assembly may comprise engaging the first intermediate module, for example an upstream end of the first intermediate module, with the upstream module, for example a downstream end of the upstream module.

Engaging the upstream module and the downstream module with the intermediate module assembly may comprise engaging each of the one or more additional intermediate modules, in turn, with its immediately upstream module until a most-downstream module of the one or more additional intermediate modules has been engaged with its immediately upstream module.

Engaging the upstream module and the downstream module with the intermediate module assembly may comprise engaging the final intermediate module, for example an upstream end of the final intermediate module, with the most-downstream module of the one or more additional intermediate modules, for example a downstream end of the most-downstream module of the one or more additional intermediate modules.

Engaging the upstream module and the downstream module with the intermediate module assembly may comprise engaging the final intermediate module, for example a downstream end of the final intermediate module, with the downstream module, for example an upstream end of the downstream module.

Engaging each of the one or more additional intermediate modules, in turn, with its immediately upstream module may comprise engaging, in turn, an upstream end of each of the one or more additional intermediate modules with its immediately upstream module, for example a downstream end of its immediately upstream module.

The method may comprise attaching a first delivery component, for example a first intra-tracheal or a first nasal delivery component, to the first gas delivery outlet. The method may comprise engaging a first subject, for example a first non-human animal, to the first delivery component.

The intermediate module assembly may comprise the second intermediate module The method may comprise attaching a second delivery component, for example a second intra-tracheal or a second nasal delivery component, to the second gas delivery outlet. The method may comprise engaging a second subject, for example a second non-human animal, with the second delivery component.

Advantageously, the method may assemble a system which can simultaneously deliver gas to at least one subject by intra-tracheal delivery and deliver gas to at least one other subject by nasal delivery.

The upstream module may comprise a first inlet port for introducing a first substance into a gas flow from the gas inlet. The method may comprise engaging a source of the first substance with the first inlet port.

The upstream module may comprise a second inlet port for introducing a second substance into a gas flow from the gas inlet. The method may comprise engaging a source of the second substance with the second inlet port.

The system comprises an exhaust unit. The method may comprise engaging an exhaust unit with the downstream module.

The system may comprise a spacer. The method may comprise engaging the spacer with the downstream module. The method may comprise engaging the exhaust unit with the spacer.

The method may comprise providing a gas flow along the gas flow path. The method may comprise providing a first delivery gas flow along the first delivery gas flow path.

The method may comprise providing the gas flow through the gas inlet at a predetermined pressure. The method may comprise providing the gas flow through the gas inlet at a pressure above atmospheric pressure, for example at a pressure which is one or both of at least 10 or 30 Pascals above atmospheric pressure and no more than 50 or 30 Pascals above atmospheric pressure. This pressure may be between 0 and 50, for example between 10 and 30, Pascals above atmospheric pressure. This pressure may drive gas flow through the system. That is, this pressure may drive one or more of the gas flow along the gas flow path, the first delivery gas flow along the first delivery gas flow path, and the second delivery gas flow along the second delivery gas flow path.

The method may comprise operating the exhaust unit to provide or allow the gas flow along the gas flow path. The method may comprise operating the exhaust unit to provide or allow the first delivery gas flow along the first delivery gas flow path.

The method may comprise introducing an anaesthetic gas into the gas flow from the gas inlet, for example through the first inlet port or the second inlet port.

The method may comprise introducing a substance into the gas flow from the gas inlet, for example through the first inlet port or the second inlet port, so as to form an aerosol.

The method may comprise determining a pressure in the system, for example using the pressure sensor described above.

The method may comprise adjusting a pressure in the system, for example using the pressure adjustment mechanism described above. The method may comprise adjusting a pressure in the system automatically, for example to keep the pressure within a predetermined pressure range, as described above.

The method may comprise adjusting a pressure in the system automatically based on feedback from the pressure sensor. The method may comprise maintaining a pressure in the system within a predetermined range. The method may comprise maintaining a pressure in the system within a predetermined range automatically based on feedback from the pressure sensor.

As noted above, the system may comprise a pressure adjustment mechanism for adjusting a pressure in the system. Thus, according to a fourth aspect, there is provided a gas delivery system for delivering gas to at least one subject, the system comprising: an upstream portion comprising a gas inlet; a downstream portion comprising a gas outlet; and an intermediate portion comprising a first gas delivery outlet. The upstream portion is upstream of the intermediate portion, and the intermediate portion is upstream of the downstream portion. A gas flow path is formed from the gas inlet of the upstream portion to the gas outlet of the downstream portion. A first gas delivery flow path is formed from the gas inlet of the upstream portion to the first gas delivery outlet of the intermediate portion. The system comprises a pressure adjustment mechanism for adjusting a pressure in the system.

Advantageously, the system according to the fourth aspect may allow a system operator to adjust the pressure in the system. This may allow the system operator to better control the gas flow rate through the system.

All features described above in relation to the pressure adjustment mechanism may be applicable to the pressure adjustment mechanism of the fourth aspect.

Features described in relation to the downstream module or the upstream module of the second aspect may be equally applicable to the downstream portion or the upstream portion of the first or fourth aspect, respectively. Similarly, features described in relation to the intermediate module assembly or one or more of the intermediate modules of the second aspect may be equally applicable to the intermediate portion of the first or fourth aspect.

Features described in relation to a system, for example the systems of the first, second or fourth aspects, may be applicable to the methods described herein, for example the method of the third aspect.

Features described in relation to any intermediate module may be applicable to any other intermediate module.

Features described in relation to a gas delivery outlet, for example the first gas delivery outlet, may be applicable to any or all other gas delivery outlets, for example the first additional gas delivery outlet, the second gas delivery outlet, and the second additional gas delivery outlet.

As used herein, the term "engaged with" may be used to mean "directly engaged with". Engaged components may be connected, or attached, to each other. Engaged components may be in contact with each other. Engagement of two components may require no additional components such as adaptors or engagement means separate to the engaged components.

As used herein, the term "longitudinal direction" may refer to a direction extending from an upstream end of a component to a downstream end of a component. Thus, in reference to an intermediate module, the term "longitudinal direction" may refer to a direction extending from the upstream end of the module to a downstream end of the module. And, in reference to the system, the term "longitudinal direction" may refer to a direction extending from the upstream module to the downstream module, for example from an upstream end of the upstream module to a downstream end of the downstream module.

The invention is defined in the claims.

Examples will now be further described with reference to the figures in which:
Figure 1 shows a first gas delivery system;
Figure 2 shows a second gas delivery system;
Figure 3 shows a third gas delivery system; and
Figure 4 shows a fourth gas delivery system.

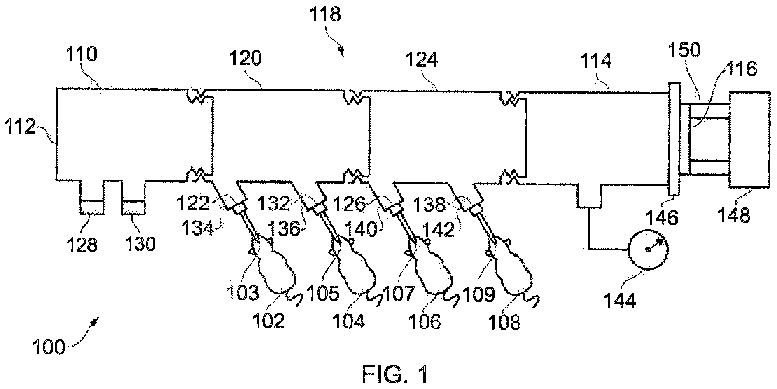

Figure 1 shows a first gas delivery system 100. The gas delivery system 100 is a modular gas delivery system for delivering gas to a plurality of subjects 102, 104, 106, 108 simultaneously. In this case, the subjects 102, 104, 106, 108 are mice. The system 100 comprises an upstream module 110 comprising a gas inlet 112, a downstream module 114 comprising a gas outlet 116, and an intermediate module assembly 118.

The intermediate module assembly 118 comprises a first intermediate module 120 comprising a first gas delivery outlet 122, and a second intermediate module 124 comprising a second gas delivery outlet 126. The intermediate module assembly 118 is engageable with, and disengageable from, the upstream module 110, and is also engageable with, and disengageable from, the downstream module 114.

In Figure 1, the upstream module 110, the downstream module 114, and the intermediate module assembly 118 are assembled into a second system configuration in which the upstream module 110 is upstream of, and engaged with, the intermediate module assembly 118, and the intermediate module assembly 118 is upstream of, and engaged with, the downstream module 114.

Assembling the system 100 into the second system configuration may comprise engaging the first intermediate module 120 with the upstream module 110, then engaging the second intermediate module 124 with the first intermediate module 120, and then engaging the downstream module 114 with the second intermediate module 124.

In the second system configuration, a gas flow path is formed from the gas inlet 112 of the upstream module 110 to the gas outlet 116 of the downstream module 114, a first gas delivery flow path is formed from the gas inlet 112 of the upstream module 110 to the first gas delivery outlet 122 of the first intermediate module 120, and a second gas delivery flow path is formed from the gas inlet 112 of the upstream module 110 to the second gas delivery outlet 126 of the second intermediate module 124.

In use, gas may be conveyed from the gas inlet 112, along the first gas delivery flow path, through the first gas delivery outlet 122, and then to the first subject 102. Such gas may be referred to as delivery gas. Simultaneously, gas may be conveyed from the gas inlet 112, along the second gas delivery flow path, through the second gas delivery outlet 126, and then to the second subject 106. Such gas may also be referred to as delivery gas. Simultaneously, gas may be conveyed from the gas inlet 112, along the gas flow path, and through the gas outlet 116 of the downstream module 114. Such gas may be referred to as exhaust gas.

The system 100 will now be described in more detail.

The upstream module 110 comprises a glass tube having an open upstream end and an open downstream end. The upstream end opposes the downstream end. An internal lumen defined by glass tube extends in a longitudinal direction between the upstream and the downstream end.

The upstream end comprises the gas inlet 112. The downstream end comprises an upstream module female engagement means which, in this case, is an internal thread located on an internal surface of the glass tube.

The upstream module 110 further comprises a first inlet port 128 for introducing a first substance into the gas flow from the gas inlet 112, and a second inlet port 130 for introducing a second substance into the gas flow from the gas inlet 112.

The first intermediate module 120 comprises a glass tube having an open upstream end and an open downstream end. The upstream end opposes the downstream end. An internal lumen defined by glass tube extends in a longitudinal direction between the upstream and the downstream end.

The upstream end comprises a first intermediate module male engagement which, in this case, is an external thread located on an external surface of the glass tube. In Figure 1, the upstream module female engagement means is engaged with the first intermediate module male engagement means. This is a reversible engagement, meaning that the upstream module 110 is engageable with, and disengageable from, the first intermediate module 120. Whilst the system is described as using internal and external threads for engagement between modules, the skilled person would understand after reading this disclosure that other means of engagement, such as snap-fit connections, clamps, or interference fits, could be used.

The downstream end comprises a first intermediate module female engagement means which, in this case, is an internal thread located on an internal surface of the glass tube.

Between the upstream end and the downstream end, the first intermediate module 120 comprises the first gas delivery outlet 122 and a first additional gas delivery outlet 132. The first additional gas delivery outlet 132 is aligned with the first gas delivery outlet 122 in the longitudinal direction. The first additional gas delivery outlet 132 is spaced from the first gas delivery outlet 122 by around 200 millimetres.

The first intermediate module 120 comprises a first gas delivery outlet structure 134 upstream of the first gas delivery outlet 122. The first gas delivery outlet structure 134 protrudes outwardly from the glass tube in a first gas delivery outlet structure direction such that an angle between the longitudinal direction and the first gas delivery outlet structure direction is about 45 degrees.

The first additional gas delivery outlet 132 comprises a similar first additional gas delivery outlet structure 136, as shown in Figure 1.

In the embodiment shown in Figure 1, the second intermediate module 124 is structurally identical to the first intermediate module 120.

Thus, the second intermediate module 124 comprises a glass tube having an open upstream end and an open downstream end. The upstream end opposes the downstream end. An internal lumen defined by glass tube extends in a longitudinal direction between the upstream and the downstream end.

The upstream end comprises a second intermediate module male engagement means which, in this case, is an external thread located on an external surface of the glass tube. In Figure 1, the first intermediate module female engagement means is engaged with the second intermediate module male engagement means. This is a reversible engagement, meaning that the first intermediate module 120 is engageable with, and disengageable from, the second intermediate module 124.

The downstream end comprises a second intermediate module female engagement means which, in this case, is an internal thread located on an internal surface of the glass tube.

Between the upstream end and the downstream end, the second intermediate module 124 comprises the second gas delivery outlet 126 and a second additional gas delivery outlet 138. The second additional gas delivery outlet 138 is aligned with the second gas delivery outlet 126 in the longitudinal direction. The second additional gas delivery outlet 138 is spaced from the second gas delivery outlet 126 by around 200 millimetres.

The second intermediate module 124 comprises a second gas delivery outlet structure 140 upstream of the first gas delivery outlet 122. The second gas delivery outlet structure 140 protrudes outwardly from the glass tube in a second gas delivery outlet structure direction such that an angle between the longitudinal direction and the second gas delivery outlet structure direction is about 45 degrees.

The second additional gas delivery outlet 138 comprises a similar second additional gas delivery outlet structure 142, as shown in Figure 1.

In the second system configuration shown in Figure 1, the first additional gas delivery outlet 132 is spaced from the second gas delivery outlet 126 by around 200 millimetres in the longitudinal direction.

The downstream module 114 comprises a glass tube having an open upstream end and an open downstream end. The upstream end opposes the downstream end. An internal lumen defined by glass tube extends in a longitudinal direction between the upstream and the downstream end. The downstream end comprises the gas outlet 116.

The upstream end comprises a downstream module male engagement means which, in this case, is an external thread located on an external surface of the glass tube. In Figure 1, the second intermediate module female engagement means is engaged with the downstream module male engagement means. This is a reversible engagement, meaning that the second intermediate module 124 is engageable with, and disengageable from, the downstream module 114.

The downstream module 114 further comprises a pressure gauge 144, which could comprise a manometer for example, for determining and displaying pressure in the system 100, and a pressure adjustment mechanism for adjusting a pressure in the system 100. The pressure adjustment mechanism is able to adjust a pressure in the system 100 during use of the system 100 to deliver gas to subjects.

The pressure adjustment mechanism comprises a pressure adjustment component 146 which, in this embodiment, comprises an iris diaphragm. The iris diaphragm defines an opening. In use, gas from the gas inlet 112 must flow through the opening to reach the gas outlet 116. Thus, the gas flow path extends through the opening. The iris diaphragm comprises a plurality of blades. The blades are moveable so as to vary a size of the opening, and thereby adjust a pressure in the system 100. Specifically, as the opening is made smaller, the pressure in the system 100 will increase provided the volumetric flow rate of gas through the system 100 is maintained.

The pressure adjustment mechanism further comprises a processor (not shown) configured to receive an input from the pressure gauge 144 relating to the pressure in the system. In use, the processor uses the input from the pressure gauge to control movement of the iris diaphragm, thereby controlling the size of the opening, and maintain a pressure in the system within a predetermined pressure range of between 10 and 30 Pascals above atmospheric pressure.

The pressure adjustment component is engageable with, and disengageable from, the downstream module. This allows the pressure adjustment component, specifically the iris diaphragm, to be removed, disassembled, and cleaned between uses of the system.

The system 100 further comprises an exhaust unit 148 for providing the gas flow from the gas inlet 112 to the gas outlet 116, and a spacer 150 for spacing the exhaust unit 148 from the downstream module 114. The spacer 150 is engageable with, and disengageable from, the downstream module 114. And the exhaust unit 148 is engageable with, and disengageable from, the spacer 150. However, it will be understood that the spacer 150 and exhaust unit 148 could be integral with the downstream module 114.

In the second system configuration shown in Figure 1, the spacer 150 is engaged with, and downstream of, the downstream module 114, and the exhaust unit 148 is engaged with, and downstream of, the spacer 150, such that the spacer 150 spaces the exhaust unit 148 from the downstream module 114 by around 50 millimetres.

The spacer 150 comprises a substantially tubular body and comprises apertures extending in a radial direction through the substantially tubular body. In the second system configuration, the exhaust unit 148 is configured to draw air from the atmosphere through the apertures in the spacer 150, and simultaneously allows gas from the gas inlet 112 to flow through the system 100. These two distinct flow paths merge within the spacer 150 and allow the pressure within the system 100 to remain stable even if the exhaust pressure were to fluctuate.

The system 100 is configured to deliver gas to the subjects 102, 104, 106, 108 by intra-tracheal delivery. The system 100 comprises an intra-tracheal delivery component 103, 105, 107, 109 for each gas delivery outlet 122, 126, 132, 138. Each intra-tracheal delivery component 103, 105, 107, 109 is engageable with, and disengageable from, each gas delivery outlet 122, 126, 132, 138. In the second system configuration shown in Figure 1, each intra-tracheal delivery component 103, 105, 107, 109 is engaged with a gas delivery outlet 122, 126, 132, 138 and is being used to intubate a mouse. Each intra-tracheal delivery component 103, 105, 107, 109 also comprises, or is engaged with, a sampler (not shown) for sampling the gas or aerosol delivered through the intra-tracheal delivery component 103, 105, 107, 109. This can be used to give an estimate of the doses of particular substances delivered through the intra-tracheal delivery component 103, 105, 107, 109.

As explained above, Figure 1 shows the system 100 in a second system configuration in which a first intermediate module 120 and a second intermediate module 124 are used. However, the system 100 is also useable in a first system configuration in which the second intermediate module 124 is not used. In the first system configuration, the downstream end of the first intermediate module 120 is engaged with the upstream end of the downstream module 114. The system 100 is also useable in a third system configuration where the system 100 comprises a third intermediate module (structurally identical to the first and second intermediate modules) and the third intermediate module is arranged between the second intermediate module 124 and the downstream module 114.

As would be understood by the skilled person after reading this disclosure, the number of intermediate modules of the system 100 may be adjusted to suit the number of subjects for which the system 100 is to be used to deliver gas.

One particular use of the system 100 is described below.

First, the system 100 is assembled into an operable system configuration such as the second system configuration shown in Figure 1.

Then, the subjects are engaged with the system 100. For the system 100 shown in Figure 1, this involves intubating mice and engaging the mice with the system 100.

Then, gas flows through the gas inlet 112 at a pressure around 10 to 30 Pascals above atmospheric pressure and the exhaust unit 148 is operated. The exhaust unit provides suction and allows gas from the gas inlet 112 of the upstream module 110 to flow through the system 100. The exhaust unit ensures that gas from the gas inlet 112 is ultimately directed to an appropriate venting location. The pressure adjustment mechanism maintains a pressure within the system 100 between 10 and 30 Pascals above atmospheric pressure.

Then a second substance, in particular a gaseous anaesthetic, is introduced into the gas flow from the gas inlet 112 through the second inlet port 130 of the upstream module. Anaesthetic gas is thus delivered to the subjects through the gas delivery outlets.

Then a first substance, in particular an atomised liquid, is introduced into the gas flow from the gas inlet 112 through the first inlet port 128 of the upstream module. The atomised liquid forms an aerosol in the system, and the aerosol is thus delivered to the subjects through the gas delivery outlets.

As would be understood by the skilled person, whilst the gaseous anaesthetic and atomised liquid in this embodiment are introduced into the gas flow through the inlet ports 128, 130 of the upstream module 110, either could be introduced into the gas flow prior to the gas flow flowing through the gas inlet 112 of the upstream module 110. For example, one could connect a liquid atomiser to the upstream module 110 upstream of the gas inlet 112 so as to atomise a liquid into the gas flow before the gas flow flows through the gas inlet 112. In this case, an aerosol may flow through the gas inlet 112.

The system 100 is operated in this manner for a predetermined time period, or until a predetermined dose of the atomised liquid has been delivered to each of the subjects. The doses delivered to the subjects can be estimated using the samplers of the intra-tracheal delivery components, as described above. The exhaust unit 148 may then be switched off and the subjects may be disengaged from the system 100 for analysis.

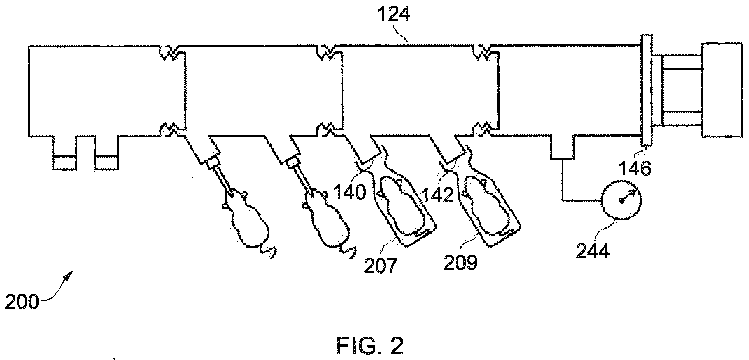

Figure 2 shows a second gas delivery system 200. The second gas delivery system 200 is similar to the first gas delivery system 100 of Figure 1 and so only the differences between the two systems are described here.

In the system 200 of Figure 2, the two of the intra-tracheal delivery components 107, 109 of the first gas delivery system 100 have been replaced by nasal delivery components 207, 209. In the second gas delivery system 200, each of the nasal delivery components 207, 209 comprises a glass bottle for accommodating a mouse.

The second intermediate module 124 has not changed. The second gas delivery outlet structure 140 and the additional second gas delivery outlet structure 142 are configured such that they can engage with an intra-tracheal delivery component, as shown in Figure 1, or a nasal delivery component, as shown in Figure 2.

Thus, the system 200 shown in Figure 2 may be used to simultaneously deliver gas to subjects by intra-tracheal delivery and by nasal delivery.

In addition, the pressure gauge 144 and associated sensor of the first gas delivery system 100 of Figure 1 have been replaced by an alternative pressure gauge 244 which is manually operated. The pressure gauge 244 determines and displays a pressure in the system 200 in real time. Based on a reading from the pressure gauge 244, a system operator is able to manually turn a dial (not shown) in order to move of the iris diaphragm of the pressure adjustment component 146, and thereby control the size of the opening and the pressure in the system 200.

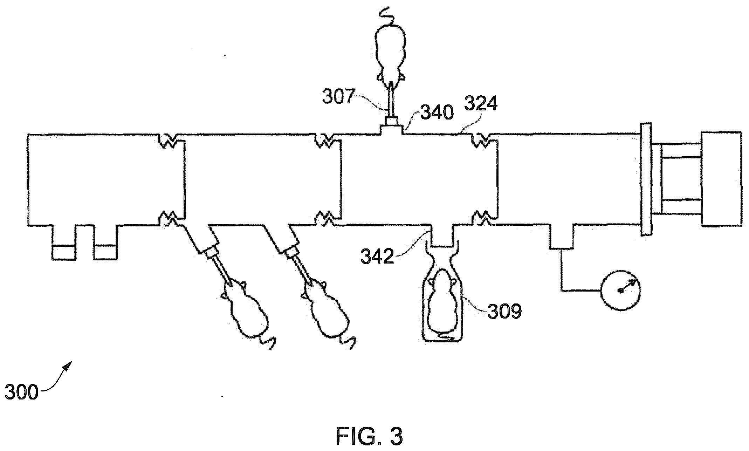

Figure 3 shows a third gas delivery system 300. The third gas delivery system 300 is structurally identical to the first gas delivery system 100 of Figure 1 except for the second intermediate module 124 and the associated intra-tracheal delivery components 107, 109 of the first gas delivery system 100. These have been replaced by an alternative second intermediate module 324, and associated alternative intra-tracheal and nasal delivery components 307, 309.

The alternative second intermediate module 324 is structurally identical to the second intermediate module 124 except in that the second gas delivery outlet structure 140 and the additional second gas delivery outlet structure 142 have been replace by an alternative second gas delivery outlet structure 340 and an alternative additional second gas delivery outlet structure 342.

The alternative second gas delivery outlet structure 340 is configured to engage specifically with the alternative intra-tracheal delivery component 307 and is directed at around 90 degrees from the longitudinal direction defined by the alternative second intermediate module 324.

The alternative additional second gas delivery outlet structure 342 is configured to engage specifically with the alternative nasal delivery component 309 and is directed at around 90 degrees from the longitudinal direction defined by the alternative second intermediate module 324.

The alternative intra-tracheal delivery component 307 is similar to the intra-tracheal delivery component 107 described in relation to Figure 1 and is for intubating a mouse.

The alternative nasal delivery component 309 is similar to the nasal delivery component 209 described in relation to Figure 2 and comprises a glass bottle for accommodating a mouse.

Thus, the system 300 shown in Figure 3 may be used to simultaneously deliver gas to subjects by intra-tracheal delivery and by nasal delivery. In particular, an individual module, the alternative second intermediate module 324, of the intermediate module assembly allows simultaneous delivery to subjects by intra-tracheal and nasal delivery.

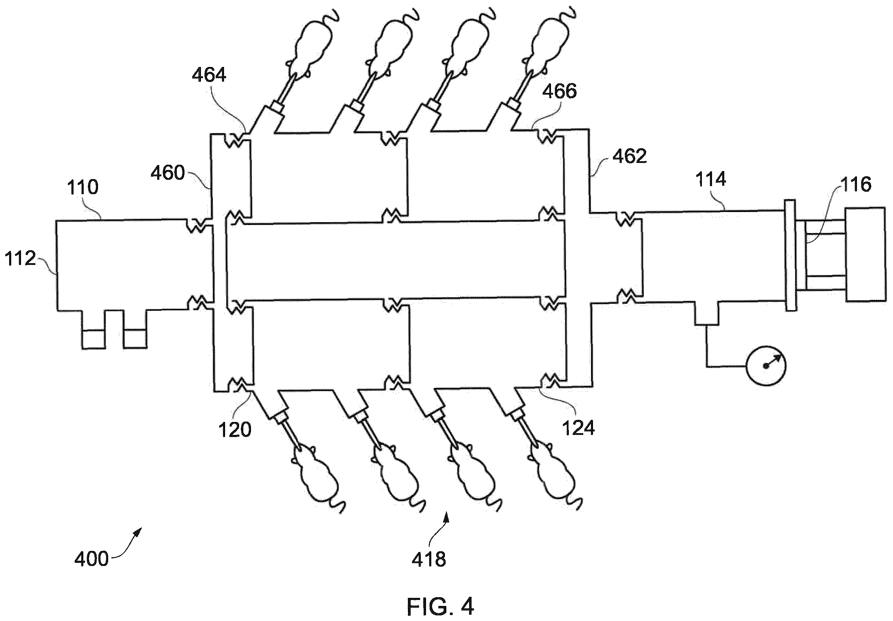

Figure 4 shows a fourth gas delivery system 400 assembled into a parallel system configuration. The fourth gas delivery system 400 is similar to the first gas delivery system 100 of Figure 1 so only the major differences between the systems 100, 400 are described here.

The intermediate module assembly 418 of the fourth gas delivery system 400 comprises a third intermediate module 464 and a fourth intermediate module 466 in addition to the first intermediate module 120 and the second intermediate module 124. In the parallel system configuration shown in Figure 4, the first intermediate module 120 and the second intermediate module 124 are arranged in parallel with the third intermediate module 464 and the fourth intermediate module 466. All of the intermediate modules 120, 124, 464, 466 are structurally identical such that they can be used interchangeably.

The fourth gas delivery system 400 comprises an upstream adaptor 460, also referred to as an upstream divider 460, positioned between the upstream module 110 and the upstream end of the intermediate module assembly 418. The upstream divider 460 is engaged with the first intermediate module 120 and the third intermediate module 464. The upstream divider 460 divides a gas flow path from the gas inlet 112 of the upstream module 110 into two flow paths. The system 400 further comprises a downstream adaptor 462, also referred to as a downstream merger 462, positioned between the downstream end of the intermediate module assembly 418 and the downstream module 114. The downstream merger 462 is engaged with the second intermediate module 124 and the fourth intermediate module 466. The downstream merger 462 merges two gas flow paths, a gas flow path from each of the two branches of the intermediate module assembly 418 arranged in parallel, into a single flow path.

In the system 400 shown in Figure 4, the upstream divider 460 is engageable with, and disengageable from, the upstream module 110 and the intermediate module assembly 418. And the downstream merger 462 is engageable with, and disengageable from, the downstream module 114 and the intermediate module assembly 418. This allows the system 400 to be operated in the second system configuration as shown in Figure 1 (by not using the adaptors 460, 462 or the third or fourth intermediate modules 464, 466), which is a series system configuration, or in the parallel system configuration shown in Figure 4.

As would be understood by the skilled person after reading this disclosure, the upstream divider 460 and the downstream merger 462 may engage with the modules of the system 400 in a similar manner to how the other modules of the system 400 engage with each other, or in a different manner, for example with snap-fit connections, clamps or interference fits.

In the fourth gas delivery system 400, two gas flow paths are defined from the gas inlet 112 of the upstream module 110 to the gas outlet 116 of the downstream module 114. The first of these gas flow paths extends through the first intermediate module 120 and the second intermediate module 124, and the second of these gas flow paths extends through the third intermediate module 464 and the fourth intermediate module 466.

In addition, delivery gas flow paths are defined from the gas inlet 112 of the upstream module 110 to each of the two gas delivery outlets of each of the four intermediate modules 120, 124, 464, 466. Thus, as shown in Figure 4, the system 400 is able to simultaneously deliver gas to eight subjects.

For the purpose of the present description and of the appended claims, except where otherwise indicated, all numbers expressing amounts, quantities, percentages, and so forth, are to be understood as being modified in all instances by the term "about". Also, all ranges include the maximum and minimum points disclosed and include any intermediate ranges therein, which may or may not be specifically enumerated herein. In this context, therefore, a number A is understood as A ± 10% of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

## Claims

1. A gas delivery system (100, 200, 300, 400) for delivering gas to at least one subject, the system comprising:
an upstream portion comprising a gas inlet (112);
a downstream portion comprising a gas outlet (116); and
an intermediate portion comprising a first gas delivery outlet (122),
wherein the upstream portion is upstream of the intermediate portion, and the intermediate portion is upstream of the downstream portion,
and wherein:
a gas flow path is formed from the gas inlet (112) of the upstream portion to the gas outlet (116) of the downstream portion; and
a first gas delivery flow path is formed from the gas inlet (112) of the upstream portion to the first gas delivery outlet (122) of the intermediate portion;
wherein the system comprises an exhaust unit (148) for providing the gas flow from the gas inlet (112) to the gas outlet (116), the exhaust unit being configured to provide suction downstream of the gas outlet.

2. A system (100, 200, 300, 400) according to claim 1, wherein the system comprises a pressure adjustment mechanism for adjusting a pressure in the system.

3. A system (100, 200, 300, 400) according to claim 2, wherein the downstream portion comprises the pressure adjustment mechanism.

4. A system (100, 200, 300, 400) according to any of claims 2 to 3, wherein the system comprises a pressure sensor for determining a pressure in the system.

5. A system (100, 200, 300, 400) according to any of 2 to 4, wherein the pressure adjustment mechanism is a manual pressure adjustment mechanism configured to allow a user to manually adjust a pressure in the system in use.

6. A system (100, 200, 300, 400) according to any of claims 2 to 4, wherein the pressure adjustment mechanism is an automatic pressure adjustment mechanism configured to automatically adjust a pressure in the system in use.

7. A system (100, 200, 300, 400) according to claim 6, when dependent on claim 4, wherein, in use, the pressure adjustment mechanism is configured maintain a pressure in the system within a predetermined pressure range based on a pressure sensed by the pressure sensor.

8. A system (100, 200, 300, 400) according to any of claims 2 to 7, wherein the pressure adjustment mechanism comprises a pressure adjustment component (146) which defines an opening, wherein the gas flow path extends through the opening.

9. A system (100, 200, 300, 400) according to claim 8, wherein the pressure adjustment component (146) comprises a plurality of blades, wherein at least one of the plurality of blades is moveable so as to vary a size of the opening.

10. A system (100, 200, 300, 400) according to claim 9, wherein the pressure adjustment component (146) is or comprises an iris diaphragm.

11. A system (100, 200, 300, 400) according to any of claims 8 to 10, when dependent on claim 4, wherein the pressure adjustment mechanism comprises a processor, wherein, in use, the processor uses an input from the pressure sensor to control movement of the pressure adjustment component (146) to maintain a pressure in the system within a predetermined pressure range.

12. A system (100, 200, 300, 400) according to any of claims 8 to 11, wherein the pressure adjustment component (146) is engageable with, and disengageable from, the downstream portion of the system.

13. A system (100, 200, 300, 400) according to any of claims 8 to 12, wherein the pressure adjustment component (146) is disassembleable and reassemblable.

14. A system (100, 200, 300, 400) according to any preceding claim , wherein the system comprises a spacer (150) for spacing the exhaust unit (148) from the downstream portion.

## Patentansprüche

1. Gasversorgungssystem (100, 200, 300, 400) zur Versorgung wenigstens eines Subjekts mit Gas, das System umfassend:
einen vorgelagerten Abschnitt, umfassend einen Gaseinlass (112);
einen nachgelagerten Abschnitt, umfassend einen Gasauslass (116); und
einen Zwischenabschnitt, umfassend einen ersten Gasabgabeauslass (122),
wobei der vorgelagerte Abschnitt dem Zwischenabschnitt vorgelagert ist und der Zwischenabschnitt dem nachgelagerten Abschnitt vorgelagert ist,
und wobei:
ein Gasströmungsweg von dem Gaseinlass (112) des vorgelagerten Abschnitts zu dem Gasauslass (116) des nachgelagerten Abschnitts gebildet wird; und
ein erster Gasabgabeströmungsweg von dem Gaseinlass (112) des vorgelagerten Abschnitts zu dem ersten Gasabgabeauslass (122) des Zwischenabschnitts gebildet wird;
wobei das System eine Abgaseinheit (148) zum Bereitstellen des Gasstroms von dem Gaseinlass (112) zu dem Gasauslass (116) umfasst, wobei die Abgaseinheit dazu ausgelegt ist, eine Saugwirkung stromabwärts des Gasauslasses bereitzustellen.

2. System (100, 200, 300, 400) nach Anspruch 1, wobei das System einen Druckeinstellmechanismus zum Einstellen eines Drucks in dem System umfasst.

3. System (100, 200, 300, 400) nach Anspruch 2, wobei der nachgelagerte Abschnitt den Druckeinstellmechanismus umfasst.

4. System (100, 200, 300, 400) nach einem der Ansprüche 2 bis 3, wobei das System einen Drucksensor zum Bestimmen eines Drucks in dem System umfasst.

5. System (100, 200, 300, 400) nach einem der Ansprüche 2 bis 4, wobei der Druckeinstellmechanismus ein manueller Druckeinstellmechanismus ist, der dazu eingerichtet ist, es einem Benutzer zu ermöglichen, bei Gebrauch einen Druck in dem System manuell einzustellen.

6. System (100, 200, 300, 400) nach einem der Ansprüche 2 bis 4, wobei der Druckeinstellmechanismus ein automatischer Druckeinstellmechanismus ist, der dazu eingerichtet ist, bei Gebrauch einen Druck in dem System automatisch einzustellen.

7. System (100, 200, 300, 400) nach Anspruch 6, falls abhängig von Anspruch 4, wobei bei Gebrauch der Druckeinstellmechanismus dazu eingerichtet ist, einen Druck in dem System innerhalb eines vorbestimmten Druckbereichs auf der Grundlage eines Drucks, der durch den Drucksensor erfasst wird, aufrechtzuerhalten.

8. System (100, 200, 300, 400) nach einem der Ansprüche 2 bis 7, wobei der Druckeinstellmechanismus eine Druckeinstellkomponente (146) umfasst, die eine Öffnung definiert, wobei sich der Gasströmungsweg durch die Öffnung erstreckt.

9. System (100, 200, 300, 400) nach Anspruch 8, wobei die Druckeinstellkomponente (146) eine Vielzahl von Lamellen umfasst, wobei wenigstens eine der Vielzahl von Lamellen bewegbar ist, um eine Größe der Öffnung zu variieren.

10. System (100, 200, 300, 400) nach Anspruch 9, wobei die Druckeinstellkomponente (146) eine Irisblende ist oder eine solche umfasst.

11. System (100, 200, 300, 400) nach einem der Ansprüche 8 bis 10, falls abhängig von Anspruch 4, wobei der Druckeinstellmechanismus einen Prozessor umfasst, wobei der Prozessor bei Gebrauch eine Eingabe von dem Drucksensor verwendet, um die Bewegung der Druckeinstellkomponente (146) zu steuern, um einen Druck in dem System innerhalb eines vorbestimmten Druckbereichs aufrechtzuerhalten.

12. System (100, 200, 300, 400) nach einem der Ansprüche 8 bis 11, wobei die Druckeinstellkomponente (146) mit dem nachgelagerten Abschnitt des Systems in Eingriff gebracht werden kann und von diesem gelöst werden kann.

13. System (100, 200, 300, 400) nach einem der Ansprüche 8 bis 12, wobei die Druckeinstellkomponente (146) zerlegbar und wieder zusammensetzbar ist.

14. System (100, 200, 300, 400) nach einem beliebigen vorangegangenen Anspruch, wobei das System einen Abstandshalter (150) zum Beabstanden der Abgaseinheit (148) von dem nachgelagerten Abschnitt umfasst.

## Revendications

1. Système de distribution de gaz (100, 200, 300, 400) destiné à distribuer du gaz à au moins un sujet, le système comprenant :
une portion amont comprenant une entrée de gaz (112) ;
une portion aval comprenant une sortie de gaz (116) ; et
une portion intermédiaire comprenant une première sortie de distribution de gaz (122),
dans lequel la portion amont est en amont de la portion intermédiaire, et la portion intermédiaire est en amont de la portion aval,
et dans lequel :
un trajet d'écoulement de gaz est formé depuis l'entrée de gaz (112) de la portion amont jusqu'à la sortie de gaz (116) de la portion aval ; et
un premier trajet d'écoulement de distribution de gaz est formé depuis l'entrée de gaz (112) de la portion amont jusqu'à la première sortie de distribution gaz (122) de la portion intermédiaire ;
dans lequel le système comprend une unité d'échappement (148) destinée à fournir l'écoulement de gaz de l'entrée de gaz (112) jusqu'à la sortie de gaz (116), l'unité d'échappement étant configurée pour fournir une aspiration en aval de la sortie de gaz.

2. Système (100, 200, 300, 400) selon la revendication 1, dans lequel le système comprend un mécanisme de réglage de pression destiné à régler une pression dans le système.

3. Système (100, 200, 300, 400) selon la revendication 2, dans lequel la portion aval comprend le mécanisme de réglage de pression.

4. Système (100, 200, 300, 400) selon l'une quelconque des revendications 2 à 3, dans lequel le système comprend un capteur de pression destiné à déterminer une pression dans le système.

5. Système (100, 200, 300, 400) selon l'une quelconque des revendications 2 à 4, dans lequel le mécanisme de réglage de pression est un mécanisme manuel de réglage de pression configuré pour permettre à un utilisateur de régler manuellement une pression dans le système lors de l'utilisation.

6. Système (100, 200, 300, 400) selon l'une quelconque des revendications 2 à 4, dans lequel le mécanisme de réglage de pression est un mécanisme automatique de réglage de pression configuré pour régler automatiquement une pression dans le système lors de l'utilisation.

7. Système (100, 200, 300, 400) selon la revendication 6, lorsqu'elle dépend de la revendication 4, dans lequel, lors de l'utilisation, le mécanisme de réglage de pression est configuré pour maintenir une pression dans le système au sein d'une plage de pressions prédéterminée sur la base d'une pression captée par le capteur de pression.

8. Système (100, 200, 300, 400) selon l'une quelconque des revendications 2 à 7, dans lequel le mécanisme de réglage de pression comprend un composant de réglage de pression (146) qui définit une ouverture, dans lequel le trajet d'écoulement de gaz s'étend à travers l'ouverture.

9. Système (100, 200, 300, 400) selon la revendication 8, dans lequel le composant de réglage de pression (146) comprend une pluralité de lames, dans lequel au moins l'une parmi la pluralité de lames est mobile de manière à faire varier une taille de l'ouverture.

10. Système (100, 200, 300, 400) selon la revendication 9, dans lequel le composant de réglage de pression (146) est, ou comprend, un diaphragme à iris.

11. Système (100, 200, 300, 400) selon l'une quelconque des revendications 8 à 10, lorsqu'elles dépendent de la revendication 4, dans lequel le mécanisme de réglage de pression comprend un processeur, dans lequel, lors de l'utilisation, le processeur utilise une entrée provenant du capteur de pression pour commander le mouvement du composant de réglage de pression (146) afin de maintenir une pression dans le système au sein d'une plage de pressions prédéterminée.

12. Système (100, 200, 300, 400) selon l'une quelconque des revendications 8 à 11, dans lequel le composant de réglage de pression (146) peut venir en prise avec la portion aval du système et en être dégagé.

13. Système (100, 200, 300, 400) selon l'une quelconque des revendications 8 à 12, dans lequel le composant de réglage de pression (146) peut être désassemblé et réassemblé.

14. Système (100, 200, 300, 400) selon une quelconque revendication précédente, dans lequel le système comprend un espaceur (150) destiné à espacer l'unité d'échappement (148) de la portion aval.
